# EUROPEAN PATENT APPLICATION

(11) **EP 3 427 687 A1**
(43) Date of publication of application: **16.01.2019**
(21) Application number: 18188673.0
(22) Date of filing: 14.05.2010
(51) Int. Cl.: A61B 90/00, A61B 34/20

(54) **AUTOMATIC REGISTRATION TECHNIQUE**

(30) Priority: 14.05.2009 US 178437 P
(62) Divisional of application: 10162897.2
(71) Applicant: Covidien LP, Mansfield, MA 02048 (US)
(72) Inventor: Averbuch, Dorian, 47261 Ramat HaSharon (IL)
(74) Representative: Maschio, Antonio

(57) **Abstract**

Registration between a digital image of a branched structure and a real-time indicator representing a location of a sensor inside the branched structure is achieved by using the sensor to "paint" a digital picture of the inside of the structure. Once enough location data has been collected, registration is achieved. The registration is "automatic" in the sense that navigation through the branched structure necessarily results in the collection of additional location data and, as a result, registration is continually refined.

## Description

### RELATED APPLICATIONS

This application claims priority to U.S. Provisional Application Serial No. 61/178,437 filed May 14, 2009 entitled Automatic Registration Technique, which is hereby incorporated herein by reference in its entirety.

### BACKGROUND OF THE INVENTION

Breakthrough technology has emerged which allows the navigation of a catheter tip through a tortuous channel, such as those found in the pulmonary system, to a predetermined target. This technology compares the real-time movement of a sensor against a three-dimensional digital map of the targeted area of the body.

Such technology is described in U.S. Patents 6,188,355; 6,226,543; 6,558,333; 6,574,498; 6,593,884; 6,615,155; 6,702,780; 6,711,429; 6,833,814; 6,974,788; and 6,996,430, all to Gilboa or Gilboa et al.; and U.S. Published Applications Pub. Nos. 2002/0193686; 2003/0074011; 2003/0216639; 2004/0249267 to either Gilboa or Gilboa et al. All of these references are incorporated herein in their entireties.

Using this technology begins with recording a plurality of images of the applicable portion of the patient, for example, the lungs. These images are often recorded using CT technology. CT images are two-dimensional slices of a portion of the patient. After taking several, parallel images, the images may be "assembled" by a computer to form a three-dimensional model, or "CT volume" of the lungs. Typically, so that all of these individual slices can be properly assembled, they are all taken while the patient is at the same point in the breathing cycle, such as maximum inhalation. In other words, the patient is instructed to take a full breath and hold it during the procedure.

The CT volume is used during the procedure as a map to the target. The user navigates a steerable probe that has a trackable sensor at its distal tip. The sensor provides the system with a real-time image of its location. However, because the image of the sensor location appears as a vector on the screen, the image has no context without superimposing the CT volume over the image provided by the sensor. The act of superimposing the CT volume and the sensor image is known as "registration."

There are various registration methods, some of which are described in the aforementioned references, and utilize a probe with a trackable sensor, as described above. For example, point registration involves selecting a plurality of points, typically identifiable anatomical landmarks, inside the lung from the CT volume and then using the sensor (with the help of an endoscope) and "clicking" on each of the corresponding landmarks in the lung. Clicking on the landmarks refers to activating a record feature on the sensor that signifies the registration point should be recorded. The recorded points are then aligned with the points in the CT volume, such that registration is achieved.

This method works well for initial registration in the central area but as the sensor is navigated to the distal portions of the lungs, the registration becomes less accurate as the distal airways are smaller.

Also, the point registration method matches a "snapshot" location of the landmarks to another "snapshot" (CT volume) of the lungs. Each snapshot is taken at different times and, potentially, at different points in the breathing cycle. Due to the dynamic nature of the lungs, the shape of the lungs during the CT scan is likely not the same as the shape of those same lungs during the procedure.

Moreover, because the user is "clicking" on the landmarks over the course of several breathing cycles, it is up to the user to approximate the timing of his clicking so that it roughly matches the point in the breathing cycle at which the CT scan was taken. This leaves much room for error. Finally, it is time consuming for the user to maneuver the sensor tip to the various landmarks.

Another example of a registration method utilizing a trackable sensor involves recording a segment of an airway and shape-match that segment to a corresponding segment in the CT volume.

This method of registration suffers similar setbacks to the point registration method, though it can be used in more distal airways because an endoscope is not required.

The registration should be conducted more than once to keep the registration updated. It may be inconvenient or otherwise undesirable to require additional registration steps from a user.

Additionally, this method requires that a good image exists in the CT volume for any given airway occupied by the sensor. If for example, the CT scan resulted in an airway shadowed by a blood vessel, for example, the registration will suffer because the shape data on that airway is compromised.

Another registration method tailored for trackable sensors is known as "Adaptive Navigation" and was developed and described in U.S. Published Application 2008/0118135 to Averbuch et al., incorporated by reference herein in its entirety. This registration technique operates on the assumption that the sensor remains in the airways at all times. The position of the sensor is recorded as the sensor is advanced, thus providing a shaped historical path of where the sensor has been. This registration method requires the development of a computer-generated and automatically or manually segmented "Bronchial Tree" (BT). The shape of the historical path is matched to a corresponding shape in the BT.

It would be desirable to develop a registration system that combines some of the advantages of each of the aforementioned registration methods. In particular, it would be desirable to develop a registration method that does not require excess training or skill on the part of the user, allows a fast and accurate initial registration, and integrates seamlessly with the aforementioned adaptive navigation technique once an initial registration has been established.

### DESCRIPTION OF THE INVENTION

Registration between a digital image of a branched structure and a real-time indicator representing a location of a sensor inside the branched structure is achieved by using the sensor to "paint" a digital picture of the inside of the structure. Once enough location data has been collected, registration is achieved. The registration is "automatic" in the sense that navigation through the branched structure necessarily results in the collection of additional location data and, as a result, registration is continually refined.

In aspects of the invention, methods and systems are provided for registering a real-time position indicator of a sensor on a probe within a branched structure to a three-dimensional model formed from previously-acquired images of said branched structure. In an aspect, a method comprises the following steps and a system is adapted to perform the following steps: moving a probe containing a location sensor within a branched structure; recording data pertaining to locations of said sensor while said sensor is moving through said branched structure; comparing a shape resulting from said data to an interior geometry of passages of said three-dimensional model of said branched structure; and determining a location correlation between said shape and said three-dimensional model based on said comparison. In another aspect, a method comprises the following steps and a system is adapted to perform the following steps: identifying non-tissue space (e.g. air filled cavities) in said three-dimensional model; moving a locatable probe through at least one lumen of said branched structure while recording position data of a location sensor in said probe; and aligning an image representing a location of said probe with an image of said three-dimensional model based on said recorded position data and an assumption that said probe remains located in non-tissue space in said branched structure. The system comprises a control unit that is adapted to perform certain of the method steps. The control unit may be software based and has a processing unit for processing code segments of a software program read from a storage device of the system. The software program is preferably stored on the storage device, typically an electronic non volatile memory chip, an optical storage disc, etc. The software program comprises said code segments for performing said steps of comparing a shape, and determining a location correlation, or aligning an image.

The present invention provides a registration technique useful for establishing at least an initial registration between a three-dimensional operating space and a computer model of that space. The technique is advantageous in that it is fast and easy to establish, and requires no special skill or training on the part of the user navigating a probe or sensor system, such as an endoscope.

For purposes of explanation, the pulmonary airways of the lungs are used herein, though one skilled in the art will realize the embodiments of the system of the present invention could be used in any body cavity or system: circulatory, digestive, pulmonary, to name a few. Additionally, if desired, the embodiments of the system of the present invention may also have non-medical application in navigating in a system using a virtual navigation interface.

The embodiments of the methods that the invention encompasses are also applicable to such body cavities or systems that are reachable without a major surgical intervention being necessary. A major surgical intervention is not included in embodiments of the invention. Surgical interventions that are potentially life threatening are excluded from the methods of embodiments of the invention. It is pointed out that many body cavities are reachable without the need for specifically trained surgeons. The method of embodiments of the invention is limited to use at such body cavities or systems. However, the system adapted for use with such methods may cover broader fields of medical or non-medical applications.

The registration technique, like that of the aforementioned adaptive navigation technique, operates on the premises that (1) the endoscope remains in the airways at all times and (2) recording the movement of a sensor on an endoscope results in a vastly greater sample set than recording discrete positions of a sensor on a stationary endoscope.

The registration method of the present invention may be referred to as "feature-based registration." When the CT scans are taken, the CT machine records each image as a plurality of pixels. When the various scans are assembled together to form a CT volume, voxels (volumetric pixels) appear and can be defined as volume elements, representing values on a regular grid in three dimensional space. Each of the voxels is assigned a number based on the tissue density Hounsfield number. This density value can be associated with gray level or color using well known window-leveling techniques.

The sensing volume of the electromagnetic field of the sensor system is also voxelized by digitizing it into voxels of a specific size compatible with the CT volume. Each voxel visited by the sensor can be assigned a value that correlates to the frequency with which that voxel is visited by the sensor. The densities of the voxels in the CT volume are adjusted according to these values, thereby creating clouds of voxels in the CT volume having varying densities. These voxel clouds or clusters thus match the interior anatomical features of the lungs.

By using a voxel-based approach, registration is actually accomplished by comparing anatomical cavity features to cavity voxels, as opposed to anatomical shapes or locations to structure shapes or locations. An advantage of this approach is that air-filled cavities are of a predictable range of densities. Air filled cavities may be identified as non-tissue space in the CT volume, which is a three-dimensional model. The locatable probe may be moved through the lumen while recording position data thereof. This allows for aligning an image representing a location of said probe with an image of said three-dimensional model based on said recorded position data and an assumption that said probe remains located in non-tissue space. When moving the probe containing a location sensor within a branched structure, data is recorded pertaining to locations of said sensor while said sensor is moving through said branched structure. Then a shape resulting from said data is compared to an interior geometry of passages of said three-dimensional model of said branched structure. This provides for determining a location correlation between said shape and said three-dimensional model based on said comparison.

Registration using the technique of the present invention is accomplished by placing an endoscope or sensor probe into the airways and continually recording its position. Doing so "paints a picture" of the airways that will, depending on the duration of the sample period and the number of airways entered during this exploration phase, fit the three-dimensional model in only one way. The more airways explored, or the more movement in any particular airway, the more accurate the initial registration.

This provides for automatic registration to happen. A navigation system is used to "paint" or draw a three dimensional image of the inside of the airways. This continues until there is enough data for a shape-matching algorithm to determine that the "painted" shape can only fit within the 3D CT volume in one place and orientation.

One particularly elegant feature of the present invention is that quite often, an initial survey of the airways is performed at the beginning of a procedure. If so, the initial survey will typically suffice to establish an initial registration. Hence, from the perspective of the user, a navigation system employing the present invention no longer requires an initial registration phase.

Another way to accomplish initial registration is to simply navigate the probe down a plurality of various airways, preferably selected in both lungs. As stated above, the more airways visited, the smaller the registration error.

Yet another way to accomplish initial registration operates with the understanding that a probe is much smaller than the larger airways, hence a single path through a large airway would result in a large error. However, if a data cloud were obtained by visiting a multitude of points in an airway, especially at the various extents of the airway, registration may be accomplished even though few airways are visited. For example, one probe for use with the present invention includes a curved distal end, others include steerable probes that may be curved at their distal ends. If the probe is inserted into a large airway with the distal end curved, it is possible to rotate the probe around its longitudinal axis while advancing or retracting the probe. Due to the curved distal end of the probe, this rotation combined with the axial movement, will result in a corkscrew that accurately "paints" the walls of the airway. Rotating the probe around its axis is an especially fast way to achieve registration.

One aspect of the present invention provides user feedback during the data collection phase. An indication will appear informing the user that accurate registration has been achieved and that the actual navigation may begin.

This represents a big leap forward in terms of ease-of-use for the users, who used to have to navigate and "mark" a large number of points in the lungs, by "clicking" on each of corresponding landmarks, in order to achieve registration.

Another aspect of the present invention provides user feedback in the form of a registration error indicator. Rather than merely providing a signal indicating that the navigation may commence, an actual registration error may be displayed. This gives the user the option of collecting additional data prior to navigation if increased accuracy is desired. The user also gets the benefit of seeing how his or her actions during the data collection phase affect the accuracy of the registration. Error measurements such as fit, spread and/or tightness may be included. Fit is a term used to describe the overall error of the match. Spread is a term that describes the width of the sample, understanding that widely spaced airway samples will typically result in a better match. Tightness describes how much the sample "cloud" may be moved around within the model airways.

Although the invention has been described in terms of particular embodiments and applications, one of ordinary skill in the art, in light of this teaching, can generate additional embodiments and modifications without departing from the spirit of or exceeding the scope of the appended patent claims. For instance, instead of using an endoscope, other elongate access devices for positioning of the sensors may be used in the system, e.g. a catheter or a needle. Accordingly, it is to be understood that the description herein is proffered by way of example to facilitate comprehension of the invention and should not be construed to limit the scope thereof as defined by the appended patent claims.

The invention is described by the following numbered paragraphs:-
1. A method of registering a real-time position indicator of a sensor on a probe within a branched structure to a three-dimensional model formed from previously-acquired images of said branched structure, comprising:
   moving a probe containing a location sensor within a branched structure,
   recording data pertaining to locations of said sensor while said sensor is moving through said branched structure,
   comparing a shape resulting from said data to an interior geometry of passages of said three-dimensional model of said branched structure, and
   determining a location correlation between said shape and said three-dimensional model based on said comparison; or
   identifying non-tissue space in said three-dimensional model,
   moving a locatable probe through at least one lumen of said branched structure while recording position data of a location sensor in said probe, and
   aligning an image representing a location of said probe with an image of said three-dimensional model based on said recorded position data and an assumption that said probe remains located in non-tissue space in said branched structure.
2. The method of paragraph 1 wherein moving a probe containing a location sensor within a branched structure comprises moving a probe in a plurality of branches of said branched structure; or wherein moving a locatable probe through at least one lumen of said branched structure while recording position data of a location sensor in said probe comprises moving a probe in a plurality of branches of said branched structure.
3. The method of paragraph 1 wherein moving a probe containing a location sensor within a branched structure comprises conducting an initial survey of said branched structure; or wherein moving a locatable probe through at least one lumen of said branched structure while recording position data of a location sensor in said probe comprises conducting an initial survey of said branched structure.
4. The method of paragraph 1 wherein recording data pertaining to locations of said sensor while said sensor is moving through said branched structure comprises obtaining a data cloud; or wherein moving a locatable probe through at least one lumen of said branched structure while recording position data of a location sensor in said probe comprises obtaining a data cloud.
5. The method of paragraph 4 wherein obtaining a data cloud comprises moving said probe or said locatable probe through a multitude of points in at least one branch of said branched structure.
6. The method of paragraphs 4 or 5, wherein moving a probe containing a location sensor within a branched structure comprises moving a probe with a curved distal end containing a location sensor within a branched structure; or wherein moving a locatable probe through at least one lumen of said branched structure while recording position data of a location sensor in said probe comprises moving a probe with a curved distal end containing a location sensor within a branched structure.
7. The method of paragraph 6 wherein obtaining a data cloud comprises rotating the probe or locatable probe around a longitudinal axis of the probe while advancing and retracting said probe.
8. The method of any of paragraphs 1 to 7 further comprising providing feedback to a user regarding registration.
9. The method of paragraph 8 wherein providing feedback to a user regarding registration comprises alerting the user that acceptably accurate registration has been achieved.
10. The method of paragraph 8 wherein providing feedback to a user regarding registration comprises displaying an actual registration error.
11. The method of paragraph 8 wherein providing feedback to a user regarding registration comprises providing fit, and/or providing spread, and/ or providing tightness.
12. A navigation system comprising a probe and a location sensor on said probe, forming a locatable probe adapted to register a real-time position indicator of a said sensor on said probe within a branched structure to a three-dimensional model formed from previously-acquired images of said branched structure,
   wherein said system is devised to record data pertaining to locations of said sensor while said sensor is moving through said branched structure, and wherein said system comprises a control unit adapted to compare a shape resulting from said data to an interior geometry of passages of said three-dimensional model of said branched structure, and determine a location correlation between said shape and said three-dimensional model based on said comparison; or
   wherein said system is configured to identify non-tissue space in said three-dimensional model, and record position data of said location sensor in said probe while said locatable probe is moving through at least one lumen of said branched structure while, and wherein said system comprises a control unit adapted to align an image representing a location of said probe with an image of said three-dimensional model based on said recorded position data and an assumption that said probe remains located in non-tissue space in said branched structure.
13. The system of paragraph 12, wherein said probe has a curved distal end having arranged said location sensor, and wherein said probe is rotatable around a longitudinal axis of the probe, such that upon rotating the probe around the longitudinal axis while advancing or retracting the probe, a corkscrew movement is achieved that provides location data of interior walls of said branched structure.
14. The system of paragraph 12 or 13, further comprising a unit adapted to provide feedback to a user regarding registration, such as for alerting the user that acceptably accurate registration has been achieved, or displaying an actual registration error.
15. The system of paragraph 14, wherein said feedback to said user regarding registration comprises providing fit, and/or providing spread, and/ or providing tightness.

## Claims

1. A method of registering a real-time position indicator within a branched structure, comprising:
moving a probe having a location sensor on said probe through a corkscrew motion within a branched structure;
obtaining location data pertaining to interior walls of said branched structure from said sensor while said probe is moving; and
utilizing a processor to register said location data pertaining to said interior walls of said branched structure with a three-dimensional model formed from previously-acquired images of said branched structure.

2. The method according to claim 1, wherein moving said probe through said corkscrew motion includes rotating said probe about a longitudinal axis of said probe and advancing or retracting said probe.

3. The method according to claim 1, wherein said probe includes a curved distal portion and wherein said location sensor is disposed on said curved distal portion of said probe

4. The method according to claim 1, further comprising providing feedback to a user regarding registration.

5. The method according to claim 4, wherein providing feedback to a user regarding registration comprises alerting the user that acceptably accurate registration has been achieved.

6. The method according to claim 4, wherein providing feedback to a user regarding registration comprises displaying an actual registration error

7. The method according to claim 4, wherein providing feedback to a user regarding registration comprises providing fit, spread, and/or tightness.

8. A method of registering a real-time position indicator within a branched structure, comprising:
identifying non-tissue space in a three-dimensional model formed from previously-acquired images of a branched structure;
moving a probe having a location sensor on said probe through a corkscrew motion within a branched structure;
obtaining location data pertaining to interior walls of said branched structure from said sensor while said probe is moving; and
aligning an image representing a location of said probe with an image of said three-dimensional model based on said recorded location data and an assumption that said probe remains located in non-tissue space in said branched structure.

9. The method according to claim 8, wherein moving said probe through said corkscrew motion includes rotating said probe about a longitudinal axis of said probe and advancing or retracting said probe.

10. The method according to claim 8, wherein said probe includes a curved distal portion and wherein said location sensor is disposed on said curved distal portion of said probe

11. The method according to claim 8, further comprising providing feedback to a user regarding registration.

12. The method according to claim 11, wherein providing feedback to a user regarding registration comprises alerting the user that acceptably accurate registration has been achieved.

13. The method according to claim 11, wherein providing feedback to a user regarding registration comprises displaying an actual registration error

14. The method according to claim 11, wherein providing feedback to a user regarding registration comprises providing fit, spread, and/or tightness.
